# EUROPEAN PATENT SPECIFICATION

(11) **EP 4 292 090 B1**
(45) Date of publication and mention of the grant of the patent: **06.05.2026**
(21) Application number: 22708822.6
(22) Date of filing: 10.02.2022
(51) Int. Cl.: G10L 25/51, G16H 40/63, G16H 40/67, G16H 50/20, G06F 40/284, G06F 21/62, G10L 13/00, G10L 15/26

(54) **SYSTEMS FOR PROCESSING VOICE AUDIO TO SEGREGATE PERSONAL HEALTH INFORMATION**
SYSTEME ZUR VERARBEITUNG VON SPRACHAUDIO ZUR AUSSONDERUNG PERSÖNLICHER GESUNDHEITSINFORMATIONEN
SYSTÈMES DE TRAITEMENT D'AUDIO VOCAL POUR SÉPARER DES INFORMATIONS DE SANTÉ PERSONNELLES

(30) Priority: 15.02.2021 US 202163149639 P
(43) Date of publication of application: 20.12.2023
(73) Proprietor: Koninklijke Philips N.V., 5656 AG Eindhoven (NL)
(72) Inventor: HAIRALAH, Sahar Bin, 5656 AG Eindhoven (NL); GUNDUMANE, Aravind, 5656 AG Eindhoven (NL)
(74) Representative: Philips Intellectual Property & Standards
(86) International application number: PCT/EP2022/053243
(87) International publication number: WO 2022/171739

(56) References cited:
- WO-A1-2019/106517
- WO-A1-2020/036467
- CN-A- 111 986 820
- US-A1- 2014 074 454
- US-A1- 2017 300 648
- US-A1- 2018 330 069

## Description

### Field of the Disclosure

The present disclosure is directed generally to methods and systems for voice processing to segregate personal health information from other information in a voice activated device.

### Background

The voice-enabled technology landscape continues to grow. Due to this growth, voice-enabled technology has begun to expand into areas related to personal health. For example, oral health is an area where voice-enabled technology may provide value to users by, for instance, coaching users on how they should brush their teeth and providing personalized, oral health related, alerts and recommendations.

However, in certain jurisdictions, oral health, as well as other types of personal health information, may be classified similar to medical data requiring a degree of privacy and security within an "data ecosystem" (that is, a collection of infrastructure, analytics, and applications used to capture and analyze data) in which this information is processed and stored. Many of the data ecosystems used by voice-activated systems, specifically in the smart home space, lack the requisite security and privacy protections to process and store personal health information.

Accordingly, there is a need for a voice processing system to identify, segregate, and securely store personal health information. As an example, it is proposed in US 2018/330069 A1 to route private/non-private health audio data to different devices in a network such that only the user who is authorized to receive the information contained in the audio data can hear the audio data.

### Summary of the Disclosure

The invention is as defined in appended independent claim 1. Preferred embodiments are set forth in the appended dependent claims.

The present disclosure is directed generally to methods and systems for voice processing to segregate personal health information from other information in a voice activated device. Broadly, the system captures voice audio spoken by a user. A local speech-to-text transcriber generates voice text based on the captured audio. A local natural language processor (NLP) then extracts one or more spoken phrases from the voice text. Based on the extracted phrases and a personal health phrase database, a machine learning classifier then classifies the voice audio as either personal health voice audio or non-personal health voice audio. The personal health voice audio is transmitted to a "personal health data ecosystem." As used herein, the term "personal health data ecosystem" generally refers to a collection of infrastructure hardware and software applications used to capture, analyze, and store data related to the personal health of a user (such as personal and family medical histories, hygiene habits, test and laboratory results, vital sign measurements, etc.). In the personal health data ecosystem, the voice audio is processed to generate a voice response conveyed to the user via one or more speakers. The non-personal health voice audio is transmitted to an alternate data ecosystem, such as a "smart home data ecosystem." As used herein, the term "smart home data ecosystem" generally refers to a collection of infrastructure hardware and software applications used to capture, analyze, and store data related to home automation (such as information related to home entertainment, HVAC, or lighting systems, etc.).

Generally, in one aspect, a system for processing voice audio is provided. The system may include a local device. According to an example, the local device may be a smart speaker.

The local device may include a local speech-to-text transcriber. The local speech-to-text transcriber may be configured to generate voice text. The voice text may be generated based on voice audio spoken by a user.

The local device may further include a local NLP. The local NLP may be configured to extract one or more spoken phrases from the voice text.

The local device may further include a machine learning classifier. The machine learning classifier may be configured to classify the voice audio as either personal health voice audio or non-personal health voice audio. The voice audio may be classified based on the one or more spoken phrases and a personal health phrase database. According to an example, the personal health phrase database may include a plurality of oral health phrases.

According to an example, the local device may further include a local transmitter. The local transmitter may be configured to wirelessly transmit personal health voice audio to a remote personal health data ecosystem. According to a further example, the local transmitter may wirelessly transmit the personal health voice audio to the remote personal health data ecosystem via the Internet.

According to an example, the local device may further include a voice redirector. The voice redirector may be configured to transmit non-personal health voice audio to a smart home data ecosystem.

According to an example, the system may further include the remote personal health data ecosystem. The remote personal health data ecosystem may include a remote receiver configured to receive personal health voice audio.

The remote personal health data ecosystem may further include a remote speech-to-text transcriber. The remote speech-to-text transcriber may be configured to generate personal health voice text. The personal health voice text may be generated based on the personal health voice audio.

The remote personal health data ecosystem may further include a remote NLP. The remote NLP may be configured to extract one or more personal health spoken phrases from the personal health voice text.

The remote personal health data ecosystem may further include a text response generator. The text response generator may be configured to generate a text response. The text response may be generated based on the one or more personal health spoken phrases.

The remote personal health data ecosystem may further include a text-to-speech translator. The text-to-speech translator may be configured to generate a voice response. The voice response may be generated based on the text response.

The remote personal health data ecosystem may further include a remote transmitter. The remote transmitter may be configured to wirelessly transmit the voice response to one or more speakers. The one or more speakers may be configured to emit the voice response. According to an example, the remote personal health data ecosystem may be further configured to transmit the voice response to the one or more speakers via the Internet.

According to an example, the system may further include an audio sensor. The audio sensor may be configured to capture the voice audio spoken by the user.

Generally, in another aspect, a system for processing voice audio is provided. The system may include an audio directing application. The audio directing application may include an audio directing speech-to-text transcriber. The audio directing speech-to-text transcriber may be configured to generate voice text. The voice text may be generated based on voice audio spoken by a user.

The audio directing application may further include an audio directing NLP. The audio directing NLP may be configured to extract one or more spoken phrases from the voice text.

The audio directing application may further include a machine learning classifier. The machine learning classifier may be configured to classify the voice audio as either personal health voice audio or non-personal health voice audio. The voice audio may be classified based on the one or more spoken phrases and personal health phrase database.

According to an example, the audio directing application may further include a voice redirector. The voice redirector may be configured to transmit non-personal health voice audio to a smart home data ecosystem.

The system may further include a remote personal health data ecosystem. The remote personal health data ecosystem may include a remote speech-to-text transcriber. The remote speech-to-text transcriber may be configured to generate personal health voice text. The personal health voice text may be generated based on the voice audio.

The remote personal health data ecosystem may further include a remote NLP. The remote NLP may be configured to extract one or more personal health spoken phrases from the personal health voice text.

The remote personal health data ecosystem may further include a text response generator. The text response generator may be configured to generate a text response. The text response may be based on the one or more personal health spoken phrases.

The remote personal health data ecosystem may further include a text-to-speech translator. The text-to-speech translator may be configured to generate a voice response. The voice response may be based on the text response.

According to an example, the system may further include a local audio sensor. The local audio sensor may be configured to capture the voice audio spoken by the user.

According to an example, the system may further include one or more local speakers. The one or more local speakers may be configured to emit the voice response generated by the text-to-speech translator of the remote personal health data ecosystem.

Generally, in another aspect, a computer based method for processing voice audio is provided. The method may include capturing, via an audio sensor, voice audio spoken by a user. The method may further include generating, via a first speech-to-text transcriber, voice text based on the voice audio. The method may further include extracting, via a first NLP, one or more spoken phrases from the voice text. The method may further include classifying, via a machine learning classifier, the voice audio as either personal health voice audio or non-personal health voice audio based on the one or more spoken phrases and personal health phrase database. The method may further include generating, via, a second speech-to-text transcriber, personal health voice text based on the voice audio. The method may further include extracting, via a second NLP, one or more personal health spoken phrases from the personal health voice text. The method may further include generating, via a text response generator, a text response based on the one or more personal health spoken phrases. The method may further include generating, via a text-to-speech translator, a voice response based on the text response. The method may further include emitting, via a speaker, the voice response. According to an example, the method may further include transmitting, via a voice redirector, non-personal health voice audio to a smart home data ecosystem.

In various implementations, a processor or controller may be associated with one or more storage media (generically referred to herein as "memory," e.g., volatile and non-volatile computer memory such as RAM, PROM, EPROM, and EEPROM, floppy disks, compact disks, optical disks, magnetic tape, etc.). In some implementations, the storage media may be encoded with one or more programs that, when executed on one or more processors and/or controllers, perform at least some of the functions discussed herein. Various storage media may be fixed within a processor or controller or may be transportable, such that the one or more programs stored thereon can be loaded into a processor or controller so as to implement various aspects as discussed herein. The terms "program" or "computer program" are used herein in a generic sense to refer to any type of computer code (e.g., software or microcode) that can be employed to program one or more processors or controllers.

It should be appreciated that all combinations of the foregoing concepts and additional concepts discussed in greater detail below (provided such concepts are not mutually inconsistent) are contemplated as being part of the inventive subject matter disclosed herein. In particular, all combinations of claimed subject matter appearing at the end of this disclosure are contemplated as being part of the inventive subject matter disclosed herein. It should also be appreciated that terminology explicitly employed herein that also may appear in any disclosure incorporated by reference should be accorded a meaning most consistent with the particular concepts disclosed herein.

These and other aspects of the various embodiments will be apparent from and elucidated with reference to the embodiment(s) described hereinafter.

### Brief Description of the Drawings

In the drawings, like reference characters generally refer to the same parts throughout the different views. Also, the drawings are not necessarily to scale, emphasis instead generally being placed upon illustrating the principles of the various embodiments.
FIG. 1 is a flow chart of a system for processing voice audio, in accordance with an example.
FIG. 2 is a schematic of a system for processing voice audio, in accordance with an example.
FIG. 3 is a schematic of a local device for processing voice audio, in accordance with an example.
FIG. 4 is a schematic of a remote personal health data ecosystem for processing voice audio, in accordance with an example.
FIG. 5 is a schematic of a further system for processing voice audio, in accordance with an example.
FIG. 6 is a schematic of an application-side of a system for processing voice audio, in accordance with an example.
FIG. 7 is a schematic of a further remote personal health data ecosystem for processing voice audio, in accordance with an example.

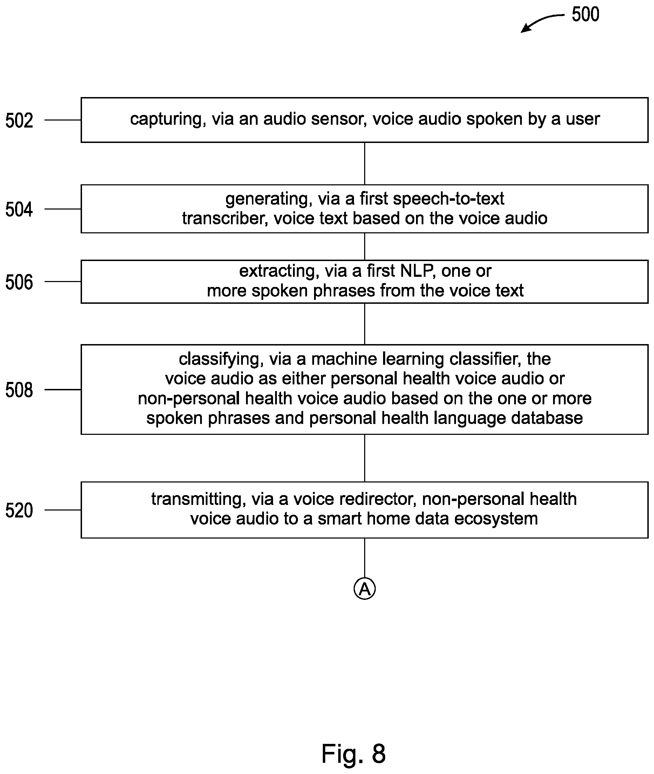

FIG. 8 is a first portion of flow chart of a method for processing voice audio, in accordance with an example.

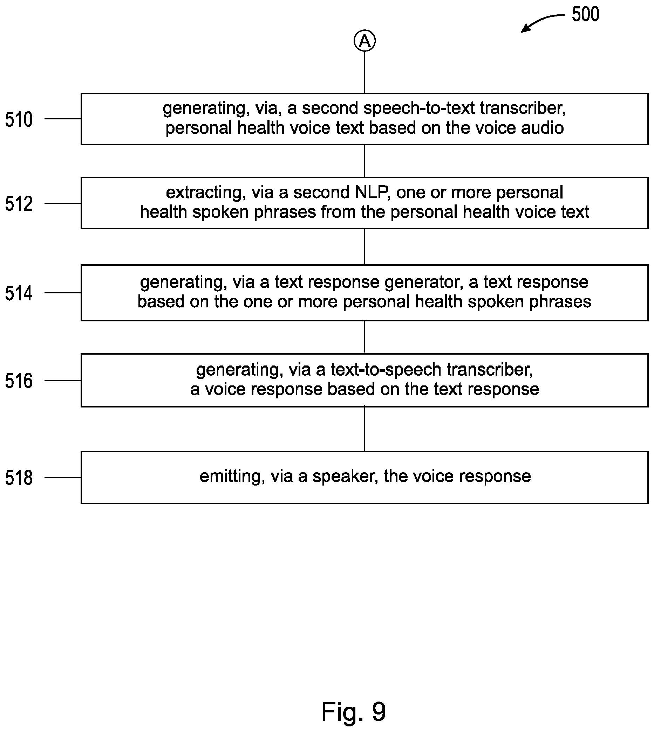

FIG. 9 is a second portion of flow chart of a method for processing voice audio, in accordance with an example.

### Detailed Description of Embodiments

The present disclosure is directed generally to methods and systems for voice processing to segregate personal health information from other information in a voice activated device. Broadly, the system captures a voice audio spoken by a user, such as, by a microphone array in a smart speaker. A local speech-to-text transcriber generates voice text based on the captured audio. A local natural language processor (NLP) then extracts one or more spoken phrases from the voice text. Based on the extracted phrases and a personal health phrase database, a machine learning classifier then classifies the voice audio as either personal health voice audio or non-personal health voice audio. The personal health voice audio is transmitted to a "personal health data ecosystem." As used herein, the term "personal health data ecosystem" generally refers to a collection of infrastructure, analytics, and applications used to capture, analyze, and store data related to the personal health of a user (such as personal and family medical histories, hyenine habits, test and laboratory results, vital sign measurements, etc.). In the personal health data ecosystem, the voice audio is processed to generate a voice response conveyed to the user via one or more speakers. The personal health data ecosystem transcribes the personal health voice audio, extracts phrases from the transcribed audio text, generates a text response based on the transcribed audio text, and translates the text response into an audio response. The non-personal health voice audio is transmitted to an alternate data ecosystem, such as a smart home data ecosystem. The personal health data ecosystem will typically be remote relative to the user, and may communicate with the user via the Internet. The personal health data ecosystem will also typically have a higher degree of security and privacy than the smart home data ecosystem.

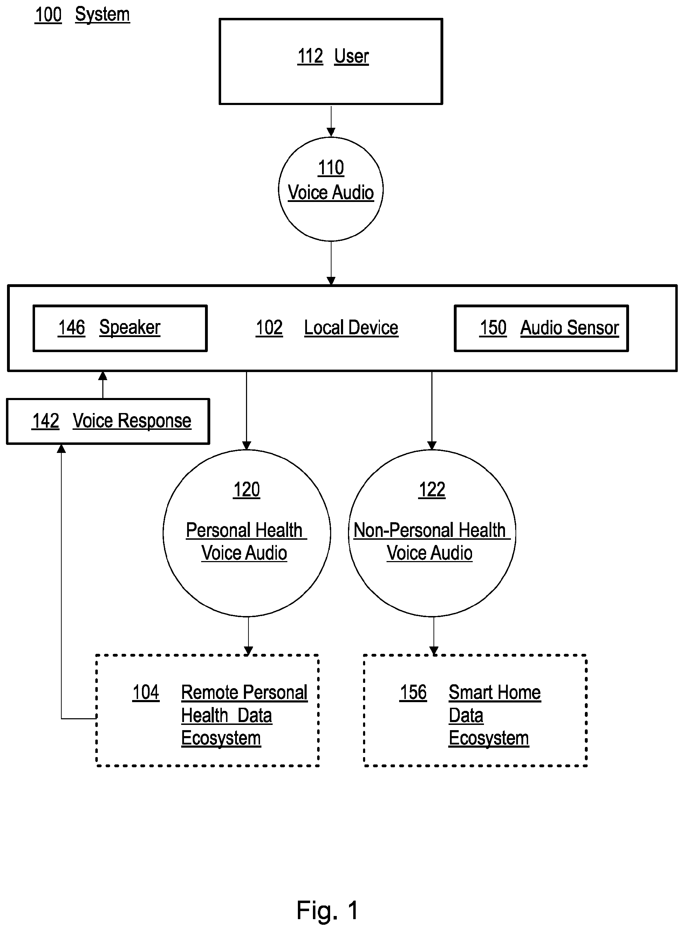

Generally, in one aspect, a system 100 for processing voice audio is provided. An overview of such a system 100 is shown in FIG. 1. According to FIG. 1, a user 112 speaks in an area proximate to a local device 102, such as a smart speaker. For example, the user 112 may ask a smart speaker "How many times did I brushed my teeth yesterday?" The local device 102 captures the voice audio 110 from the user via an audio sensor 150, such as a microphone array. The local device 102 analyzes the voice audio 110, and classifies the voice audio 110 as either personal health voice audio 120 or non-personal health voice audio 122. The local device 102 directs the personal health voice audio to a secure, remote personal health data ecosystem 104. The remote personal health data ecosystem 104 processes the personal health voice audio 120 to generate a voice response 142. For example, the voice response 142 may be an audio file answering the user's 112 question, such as "You brushed your teeth twice yesterday." The voice response is transmitted to the local device 102 and emitted by speaker 146 for the user 112 to hear. Alternatively, if the voice audio 110 is classified as non-personal health voice audio 122, this audio 122 is directed to a standard, less secure smart home data ecosystem 156 for further processing and/or storage.

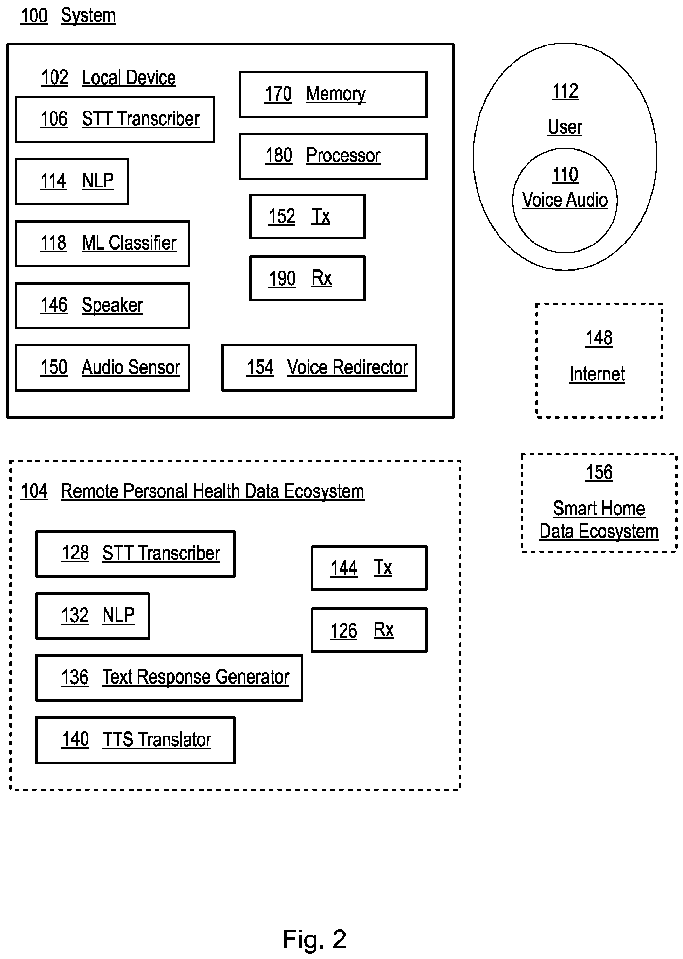

As described above, and as shown in FIG. 2, the system 100 may include a local device 102. According to an example, the local device 102 may be a smart speaker. Alternatively, the local device 102 may be a personal care device, such as a smart toothbrush. The local device 102 may also be a smart phone, personal computer, or any other device capable of processing audio data. The local device may also include a memory 170 for data storage, a processor 180 for data processing, a transmitter 152 for wireless data transmission, and a receiver 190 for wireless data reception. This hardware may be rearranged and/or reconfigured internally or externally relative to the local device 102 as appropriate. For example, the transmitter 152 and the receiver 190 may be combined as a transceiver.

According to an example, the system 100 may further include an audio sensor 150. The audio sensor 150 may be configured to capture the voice audio 110 spoken by the user 112. The audio sensor 150 may include one or more microphones, such as a microphone array. In the example of a smart speaker or a smart phone, the audio sensor 150 may be embedded in the local device 102. Alternatively, the audio sensor 150 may be external to the local device 102, such that it conveys captured voice audio to the local device 102 via a wired or wireless connection.

According to an example, the system 100 may further include one or more speakers 146. The speakers 146 may be configured to emit a voice response 142 generated by the remote personal health data ecosystem 104. In the example of a smart speaker or a smart phone, the speakers 146 may be embedded in the local device 102. Alternatively, the speakers 146 may be external to the local device 102.

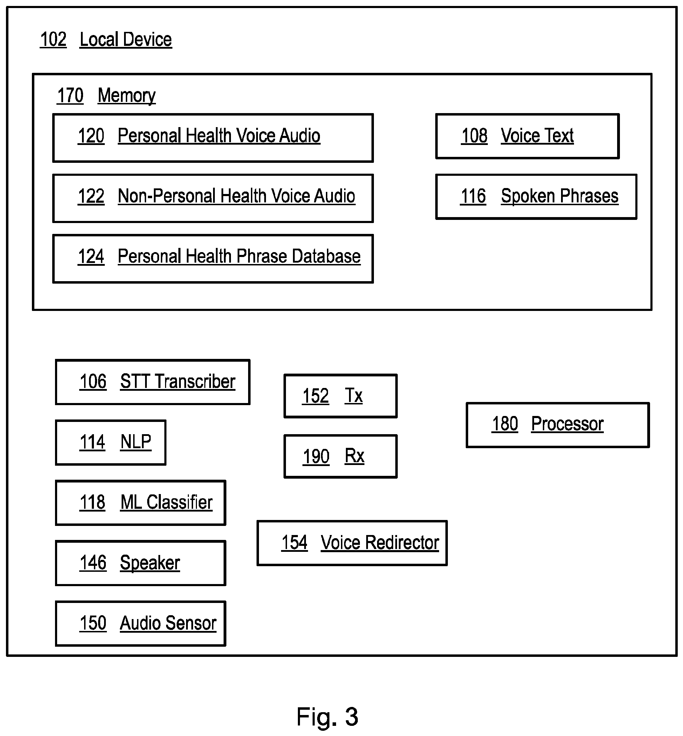

With reference to FIGS. 2 and 3, the local device 102 includes a local speech-to-text transcriber 106. The local speech-to-text transcriber 106 is configured to generate voice text 108. The voice text 108 is generated based on voice audio 110 spoken by a user 112 and captured by the audio sensor 150. In a preferred embodiment, the voice text 108 is a string of characters representing every word and/or partial word in the voice audio 110 spoken by the user 112. For example, the voice text 108 may read "Tell me about my brushing performance for the past seven days." In an alternative example, the voice text 108 may be a factual statement, rather than an inquiry, such as "One of my bottom front teeth hurts."

With further reference to FIGS. 2 and 3, the local device 102 further includes a local NLP 114. The local NLP 114 is configured to extract one or more spoken phrases 116 from the voice text 108. For example, if the voice text 108 reads "Tell me about my brushing performance for the past seven days," the local NLP 114 may extract "brushing performance" as a spoken phrase 116. As these spoken phrases 116 will be used by the machine learning classifier, described below, to classify the voice audio as personal health 120 or non-personal health 122 voice audio, in a preferred example, the local NLP 114 extracts phrases which may be probative of whether the voice audio 110 relates to personal health. A spoken phrase 116 may be a string of words, or even a single word.

With further reference to FIGS. 2 and 3, the local device 102 further includes a machine learning classifier 118. The machine learning classifier 118 is configured to classify the voice audio 110 as either personal health voice audio 120 or non-personal health voice audio 122. This classification is a crucial part of the system 100, as this classification dictates where the captured voice audio 110 is transmitted for processing and/or storage. Personal health voice audio 120 may be transmitted to a secure, remote personal health data ecosystem for processing, while non-personal health voice audio is transmitted to a less secure data ecosystem, such as a smart home data ecosystem 156. Personal health voice audio 120 may be any captured voice audio 110 pertaining to personal health information, including physical and mental health. For example, personal health voice audio 120 may relate to the oral health of the user 112.

The voice audio 110 is classified based on the one or more spoken phrases 116 and a personal health phrase database 124. As described above, the one or more spoken phrases 116 are extracted from the voice audio 110 by the local NLP 114. The personal health phrase databased 124 includes a plurality of phrases related to personal health. According to an example, the personal health phrase database 124 may include a plurality of oral health phrases. In this example, the personal health phrase database 124 may include phrases such as "toothache", "dental hygiene", "loose tooth", and/or "filling fell out". The machine learning classifier 118 trains to identify personal health voice audio 120 by analyzing the entries of the personal health phrase database 124. Through this training process, the machine learning classifier 118 learns to identify personal health voice audio 120 even when the voice audio 110 does not contain spoken phrases 116 exactly matching entries in the personal health phrase database 124.

According to an example, the local device 102 may further include a local transmitter 152. The local transmitter 152 may be configured to wirelessly transmit personal health voice audio 120 to a remote personal health data ecosystem 104. According to a further example, the local transmitter 152 may wirelessly transmit the personal health voice audio 120 to the remote personal health data ecosystem 104 via the Internet 148. Further, all or a portion of the remote personal health data ecosystem 104 may utilize cloud computing for data processing and/or storage.

According to an example, the local device 102 may further include a voice redirector 154. The voice redirector 154 may be configured to transmit the non-personal health voice audio 122 to a smart home data ecosystem 156. For example, the non-personal health voice audio 122 may contain a command or inquiry regarding entertainment, HVAC, or lighting systems. To ensure non-personal health voice audio 122 is received by the smart home data ecosystem 156, as opposed to the remote personal health data ecosystem 104, the voice redirector 154 may transmit the non-personal health voice audio 122 at a different frequency than the personal health voice audio 124. Alternatively, the voice redirector 154 may encode the non-personal health voice audio 122 with identifying information corresponding to the desired recipient ecosystem. The voice redirector 154 may transmit the non-personal health voice audio 122 via the local transmitter 152.

The system further includes the remote personal health data ecosystem 104. The remote personal health data ecosystem 104 may be generally configured to receive data related to personal health and generate a corresponding voice response 142. For example, if the remote personal health data ecosystem 104 receives audio data of a individual asking, "How many times did I brush my teeth yesterday?" the ecosystem 104 may generate a voice response 142 in the form of an audio file of a computer generated voice stating "Yesterday, you brushed your teeth twice." More detail regarding this processing may be found below. This voice response may be at least temporarily stored in memory 172 prior to transmission to the local device 102 so that the user 112 may hear the voice response 142 emitted by the speakers 146.

A data ecosystem includes a collection of infrastructure hardware and software applications used to process and/or store data. For example, a data ecosystem could include one or more servers, personal computers, mobile devices, storage drives, and other hardware. The data ecosystem also includes one or more software applications for processing the data received by the hardware. The data ecosystem may also employ various security measures, such as data encryption. The strength of the security measures may relate to the sensitivity of the data being processed and/or stored. In our case, the personal health information conveyed by the personal health voice audio 120 requires a high degree of security for both legal and ethical reasons. Non-personal health related voice audio 122 may require a lower degree of security. Hardware of the remote data ecosystem 104 may be located in a different geographical area from the user 112, such as a different neighborhood, city, state, or country. Accordingly, the system 100 will at least partially rely on the Internet 148 to transmit data to and receive data from the remote personal health data ecosystem 104. The dashed lines of remote personal health data ecosystem 104 and smart home data ecosystem 156 are meant to convey the fluid nature of the data ecosystems, rather than the rigid structure of a discrete device, such as local device 102.

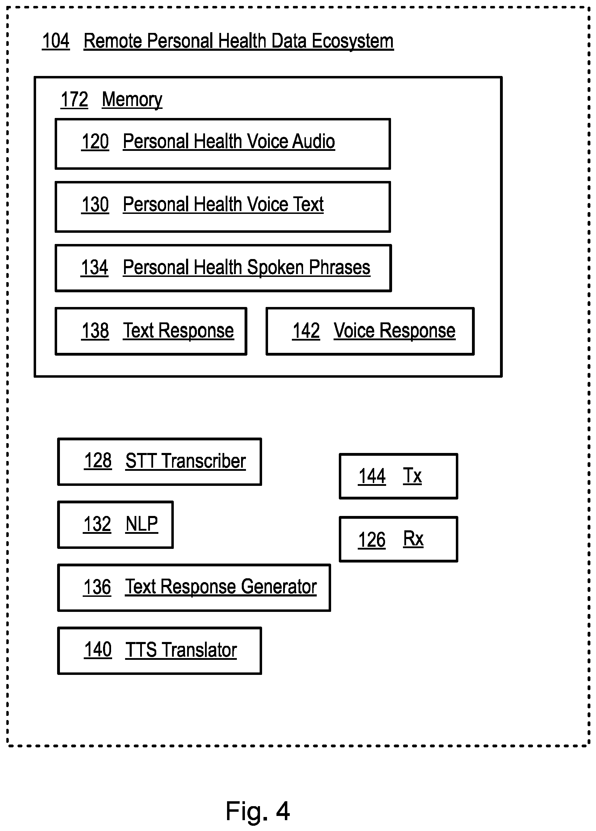

With reference to FIGS. 2 and 4, the remote personal health data ecosystem 104 includes a remote receiver 126. The remote receiver 126 is configured to receive the personal health voice audio 104 transmitted by the local device 102.

With further reference to FIGS. 2 and 4, the remote personal health data ecosystem 104 further includes a remote speech-to-text transcriber 128. The remote speech-to-text transcriber 128 is configured to generate personal health voice text 130. The personal health voice text 130 is generated based on the personal health voice audio 120. The remote speech-to-text transcriber 128 may operate similarly to the local speech-to-text transcriber 106.

With further reference to FIGS. 2 and 4, the remote personal health data ecosystem 104 further includes a remote NLP 132. The remote NLP 132 is configured to extract one or more personal health spoken phrases 134 from the personal health voice text 130. The remote NLP 132 may operate similarly to the local NLP 114.

With further reference to FIGS. 2 and 4, the remote personal health data ecosystem 104 further includes a text response generator 136. The text response generator 136 is configured to generate a text response 138. The text response may be an electronic file containing a string of characters,

The text response 138 is generated based on the one or more personal health spoken phrases 134. For example, when receiving personal health spoken phrases 134 "How many times", "brush my teeth", and "yesterday", the text response generator 138 may generate a string of characters reading "twice" or "Yesterday, you brushed your teeth twice". The text response generator 138 may access a database of personal health information of the user 112 to generate an accurate text response 138. This information may be collected from a number of sources, including various smart devices. For example, a smart toothbrush may transmit information regarding the brushing habits of the user 112 to the remote personal health data ecosystem 104 for storage in the user's 112 personal health information database. Further, the text response generator 136 may generate the text response 138 via a machine learning engine.

The remote personal health data ecosystem 104 further includes a text-to-speech translator 140. The text-to-speech translator 140 is configured to generate a voice response 142. The voice response 142 is generated based on the text response 138. For example, if the text response 138 is a string of characters reading "twice", the voice response 142 may be an audio file of a computer-generated voice saying the word "twice".

The remote personal health data ecosystem 104 further includes a remote transmitter 144. The remote transmitter 144 is configured to wirelessly transmit the voice response 142 to one or more speakers 146. The one or more speakers 146 are configured to emit the voice response 142. According to an example, the remote personal health data ecosystem 104 may be further configured to transmit the voice response 142 to the one or more speakers 146 via the Internet 148. The speakers 146 may be embedded in the local device 102, or they may be external to the local device 102. Regardless, the speakers 146 should be positioned such that the user 112 will be able to hear the emitted voice response 142.

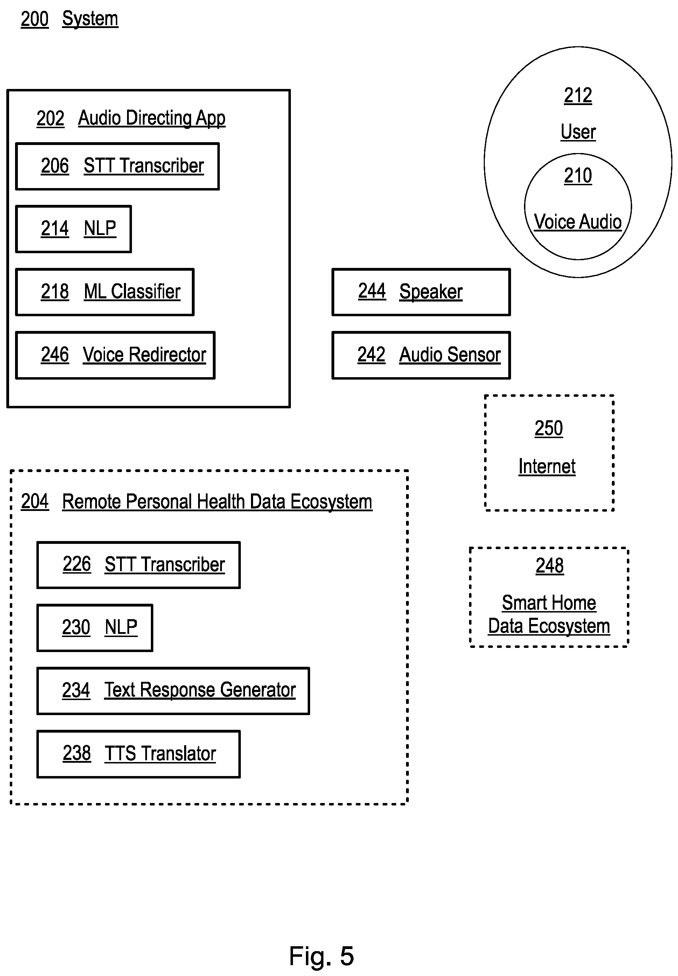

Generally, in another aspect, and with reference to FIG. 5, a system 200 for processing voice audio is provided. This system 200 is similar to the previously described system 100, with the primary difference being that hardware local to the user 212 is used primarily to capture the voice audio 210 spoken by the user 212, and to subsequently emit a voice response 240 for the user 212 via one or more speakers 244. Rather, the initial processing, classification, and directing of the voice audio 110 is performed remotely via a software application, such as a mobile app. The mobile app may process and/or store data via cloud computing or other remote computing processes. The processed voice audio 210 is subsequently directed to either a remote personal health data ecosystem 204 or smart home data ecosystem 248.

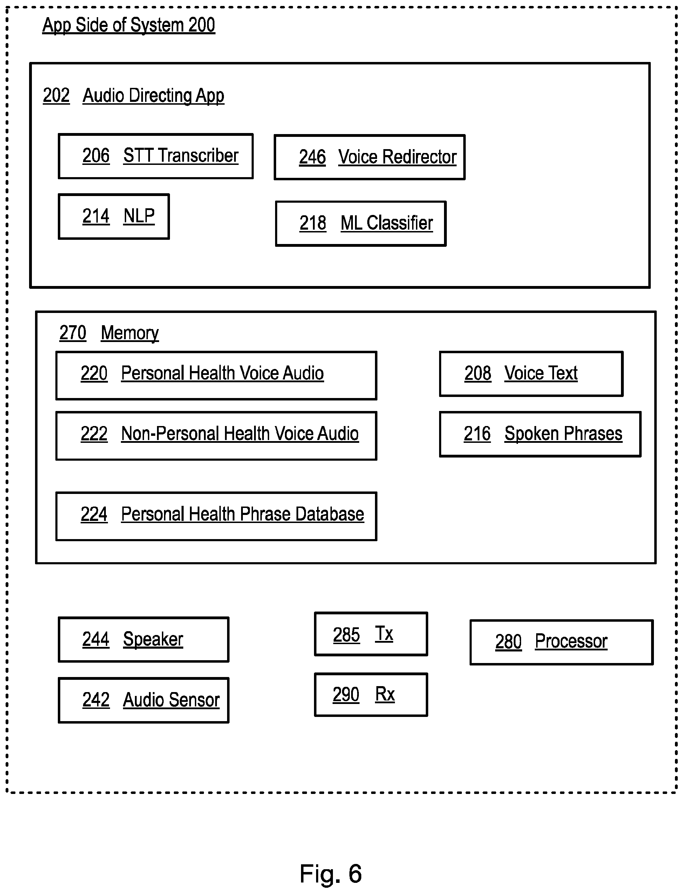

With reference to FIGS. 5 and 6, the system may include an audio directing application 202. As described above, the aspects of the audio directing application 202 may process and/or store the voice audio 210 remotely, such as by cloud computing. FIG. 6 shows the portion of system 200 application utilized by the audio directing application 202, including memory 270, processor 280, transmitter 285, receiver 290, audio sensor 242, and speaker 244. The audio directing app 202 may be accessible via a smart device, such as a smartphone, which may also include one or more of the memory 270, processor 280, transmitter 285, receiver 290, audio sensor 242, and speaker 244. Alternatively, the audio sensor 242 and/or speaker 244 may be standalone devices.

According to an example, and as described above the system 200 may further include a local audio sensor 242. The local audio sensor 242 may be configured to capture the voice audio spoken by the user. The local audio sensor 242 may be a microphone array. The local audio sensor 242 may be embedded in a smart device running the audio directing app 202. Alternatively, the local audio sensor 242 may be a standalone device.

With reference to FIGS. 5 and 6, the audio directing application 202 may include an audio directing speech-to-text transcriber 206. The audio directing speech-to-text transcriber 206 may be configured to generate voice text 208. The voice text 208 may be generated based on voice audio 210 spoken by a user 212. The audio directing application 202 may further include an audio directing NLP 214. The audio directing NLP 214 may be configured to extract one or more spoken phrases 216 from the voice text 208. The audio directing application 202 may further include a machine learning classifier 218. The machine learning classifier 218 may be configured to classify the voice audio 210 as either personal health voice audio 220 or non-personal health voice audio 222. The voice audio 210 may be classified based on the one or more spoken phrases 216 and personal health phrase database 224. According to an example, the audio directing application 202 may further include a voice redirector 246. The voice redirector 246 may be configured to transmit non-personal health voice audio 222 to a smart home data ecosystem 248.

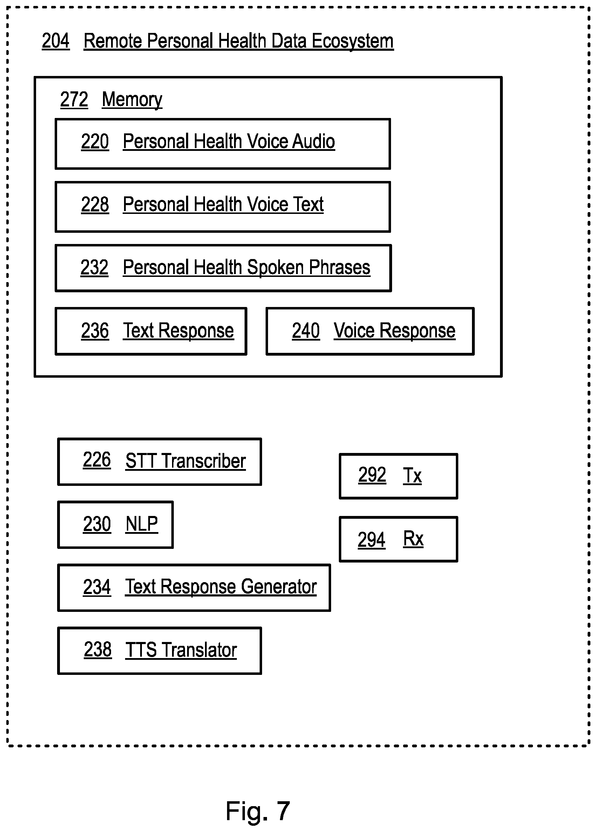

With reference to FIGS. 5 and 7, the system 200 may further include a remote personal health data ecosystem 204. The remote personal health data ecosystem 204 may include a remote speech-to-text transcriber 226. The remote speech-to-text transcriber 226 may be configured to generate personal health voice text 228. The personal health voice text 228 may be generated based on the voice audio 210. The remote personal health data ecosystem 204 may further include a remote NLP 230. The remote NLP 230 may be configured to extract one or more personal health spoken phrases 232 from the personal health voice text 228. The remote personal health data ecosystem 204 may further include a text response generator 234. The text response generator 234 may be configured to generate a text response 236. The text response 236 may be based on the one or more personal health spoken phrases 232. The remote personal health data ecosystem 204 may further include a text-to-speech translator 238. The text-to-speech translator 238 may be configured to generate a voice response 240. The voice response 240 may be based on the text response 236.

According to an example, the system 200 may further include one or more local speakers 244. The one or more local speakers 244 may be configured to emit the voice response 240 generated by the text-to-speech translator 238 of the remote personal health data ecosystem 204.

Generally, in another aspect, a computer based method 500 for processing voice audio is provided. The method 500 may include capturing 502, via an audio sensor, voice audio spoken by a user. The method 500 may further include generating 504, via a first speech-to-text transcriber, voice text based on the voice audio. The method 500 may further include extracting 506, via a first NLP, one or more spoken phrases from the voice text. The method 500 may further include classifying 508, via a machine learning classifier, the voice audio as either personal health voice audio or non-personal health voice audio based on the one or more spoken phrases and personal health phrase database. The method 500 may further include generating 510, via, a second speech-to-text transcriber, personal health voice text based on the voice audio. The method 500 may further include extracting 512, via a second NLP, one or more personal health spoken phrases from the personal health voice text. The method 500 may further include generating 514, via a text response generator, a text response based on the one or more personal health spoken phrases. The method 500 may further include generating 516, via a text-to-speech translator, a voice response based on the text response. The method 500 may further include emitting 518, via a speaker, the voice response. According to an example, the method 500 may further include transmitting 520, via a voice redirector, non-personal health voice audio to a smart home data ecosystem.

All definitions, as defined and used herein, should be understood to control over dictionary definitions, definitions in documents incorporated by reference, and/or ordinary meanings of the defined terms.

The indefinite articles "a" and "an," as used herein in the specification and in the claims, unless clearly indicated to the contrary, should be understood to mean "at least one."

The phrase "and/or," as used herein in the specification and in the claims, should be understood to mean "either or both" of the elements so conjoined, i.e., elements that are conjunctively present in some cases and disjunctively present in other cases. Multiple elements listed with "and/or" should be construed in the same fashion, i.e., "one or more" of the elements so conjoined. Other elements may optionally be present other than the elements specifically identified by the "and/or" clause, whether related or unrelated to those elements specifically identified.

As used herein in the specification and in the claims, "or" should be understood to have the same meaning as "and/or" as defined above. For example, when separating items in a list, "or" or "and/or" shall be interpreted as being inclusive, i.e., the inclusion of at least one, but also including more than one, of a number or list of elements, and, optionally, additional unlisted items. Only terms clearly indicated to the contrary, such as "only one of" or "exactly one of," or, when used in the claims, "consisting of," will refer to the inclusion of exactly one element of a number or list of elements. In general, the term "or" as used herein shall only be interpreted as indicating exclusive alternatives (i.e. "one or the other but not both") when preceded by terms of exclusivity, such as "either," "one of," "only one of," or "exactly one of."

As used herein in the specification and in the claims, the phrase "at least one," in reference to a list of one or more elements, should be understood to mean at least one element selected from any one or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase "at least one" refers, whether related or unrelated to those elements specifically identified.

It should also be understood that, unless clearly indicated to the contrary, in any methods claimed herein that include more than one step or act, the order of the steps or acts of the method is not necessarily limited to the order in which the steps or acts of the method are recited.

In the claims, as well as in the specification above, all transitional phrases such as "comprising," "including," "carrying," "having," "containing," "involving," "holding," "composed of," and the like are to be understood to be open-ended, i.e., to mean including but not limited to. Only the transitional phrases "consisting of" and "consisting essentially of" shall be closed or semi-closed transitional phrases, respectively.

The above-described examples of the described subject matter can be implemented in any of numerous ways. For example, some aspects may be implemented using hardware, software or a combination thereof. When any aspect is implemented at least in part in software, the software code can be executed on any suitable processor or collection of processors, whether provided in a single device or computer or distributed among multiple devices/computers.

The present disclosure may be implemented as a system, a method, and/or a computer program product at any possible technical detail level of integration. The computer program product may include a computer readable storage medium (or media) having computer readable program instructions thereon for causing a processor to carry out aspects of the present disclosure.

The computer readable storage medium can be a tangible device that can retain and store instructions for use by an instruction execution device. The computer readable storage medium may be, for example, but is not limited to, an electronic storage device, a magnetic storage device, an optical storage device, an electromagnetic storage device, a semiconductor storage device, or any suitable combination of the foregoing. A non-exhaustive list of more specific examples of the computer readable storage medium includes the following: a portable computer diskette, a hard disk, a random access memory (RAM), a read-only memory (ROM), an erasable programmable read-only memory (EPROM or Flash memory), a static random access memory (SRAM), a portable compact disc read-only memory (CD-ROM), a digital versatile disk (DVD), a memory stick, a floppy disk, a mechanically encoded device such as punch-cards or raised structures in a groove having instructions recorded thereon, and any suitable combination of the foregoing. A computer readable storage medium, as used herein, is not to be construed as being transitory signals per se, such as radio waves or other freely propagating electromagnetic waves, electromagnetic waves propagating through a waveguide or other transmission media (e.g., light pulses passing through a fiber-optic cable), or electrical signals transmitted through a wire.

Computer readable program instructions described herein can be downloaded to respective computing/processing devices from a computer readable storage medium or to an external computer or external storage device via a network, for example, the Internet, a local area network, a wide area network and/or a wireless network. The network may comprise copper transmission cables, optical transmission fibers, wireless transmission, routers, firewalls, switches, gateway computers and/or edge servers. A network adapter card or network interface in each computing/processing device receives computer readable program instructions from the network and forwards the computer readable program instructions for storage in a computer readable storage medium within the respective computing/processing device.

Computer readable program instructions for carrying out operations of the present disclosure may be assembler instructions, instruction-set-architecture (ISA) instructions, machine instructions, machine dependent instructions, microcode, firmware instructions, state-setting data, configuration data for integrated circuitry, or either source code or object code written in any combination of one or more programming languages, including an object oriented programming language such as Smalltalk, C++, or the like, and procedural programming languages, such as the "C" programming language or similar programming languages. The computer readable program instructions may execute entirely on the user's computer, partly on the user's computer, as a standalone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider). In some examples, electronic circuitry including, for example, programmable logic circuitry, field-programmable gate arrays (FPGA), or programmable logic arrays (PLA) may execute the computer readable program instructions by utilizing state information of the computer readable program instructions to personalize the electronic circuitry, in order to perform aspects of the present disclosure.

Aspects of the present disclosure are described herein with reference to flowchart illustrations and/or block diagrams of methods, apparatus (systems), and computer program products according to examples of the disclosure. It will be understood that each block of the flowchart illustrations and/or block diagrams, and combinations of blocks in the flowchart illustrations and/or block diagrams, can be implemented by computer readable program instructions.

The computer readable program instructions may be provided to a processor of a, special purpose computer, or other programmable data processing apparatus to produce a machine, such that the instructions, which execute via the processor of the computer or other programmable data processing apparatus, create means for implementing the functions/acts specified in the flowchart and/or block diagram block or blocks. These computer readable program instructions may also be stored in a computer readable storage medium that can direct a computer, a programmable data processing apparatus, and/or other devices to function in a particular manner, such that the computer readable storage medium having instructions stored therein comprises an article of manufacture including instructions which implement aspects of the function/act specified in the flowchart and/or block diagram or blocks.

The computer readable program instructions may also be loaded onto a computer, other programmable data processing apparatus, or other device to cause a series of operational steps to be performed on the computer, other programmable apparatus or other device to produce a computer implemented process, such that the instructions which execute on the computer, other programmable apparatus, or other device implement the functions/acts specified in the flowchart and/or block diagram block or blocks.

The flowchart and block diagrams in the Figures illustrate the architecture, functionality, and operation of possible implementations of systems, methods, and computer program products according to various examples of the present disclosure. In this regard, each block in the flowchart or block diagrams may represent a module, segment, or portion of instructions, which comprises one or more executable instructions for implementing the specified logical function(s). In some alternative implementations, the functions noted in the blocks may occur out of the order noted in the Figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions.

While various examples have been described and illustrated herein, those of ordinary skill in the art will readily envision a variety of other means and/or structures for performing the function and/or obtaining the results and/or one or more of the advantages described herein, and each of such variations and/or modifications is deemed to be within the scope of the examples described herein. More generally, those skilled in the art will readily appreciate that all parameters, dimensions, materials, and configurations described herein are meant to be exemplary and that the actual parameters, dimensions, materials, and/or configurations will depend upon the specific application or applications for which the teachings is/are used. Those skilled in the art will recognize, or be able to ascertain using no more than routine experimentation, many equivalents to the specific examples described herein. It is, therefore, to be understood that the foregoing examples are presented by way of example only and that, within the scope of the appended claims and equivalents thereto, examples may be practiced otherwise than as specifically described and claimed. Examples of the present disclosure are directed to each individual feature, system, article, material, kit, and/or method described herein. In addition, any combination of two or more such features, systems, articles, materials, kits, and/or methods, if such features, systems, articles, materials, kits, and/or methods are not mutually inconsistent, is included within the scope of the present disclosure.

## Claims

1. A system (100) for processing voice audio comprising a local device (102), the local device (102) comprising:
a local speech-to-text transcriber (106) configured to generate voice text (108) based on voice audio (110) spoken by a user (112);
a local natural language processor (NLP) (114) configured to extract one or more spoken phrases (116) from the voice text (108); and
a machine learning classifier (118) configured to classify the voice audio (110) as either personal health voice audio (120) or non-personal health voice audio (122) based on the one or more spoken phrases (116) and a personal health phrase database (124),
wherein the system (100) further comprises a remote personal health data ecosystem (104), the remote personal health data ecosystem (104) comprising:
a remote receiver (126) configured to receive the personal health voice audio (120);
a remote speech-to-text transcriber (128) configured to generate personal health voice text (130) based on the personal health voice audio (120);
a remote NLP (132) configured to extract one or more personal health spoken phrases (134) from the personal health voice text (130);
a text response generator (136) configured to generate a text response (138) based on the one or more personal health spoken phrases (134);
a text-to-speech translator (140) configured to generate a voice response (142) based on the text response (138); and
a remote transmitter (144) configured to wirelessly transmit the voice response (142) to one or more speakers (146) configured to emit the voice response (142).

2. The system (100) of claim 1, wherein the remote personal health data ecosystem (104) is further configured to transmit the voice response (142) to the one or more speakers (146) via the Internet (148).

3. The system (100) of claim 1, wherein the system (100) further comprises an audio sensor (150) configured to capture the voice audio (110) spoken by the user (112).

4. The system (100) of claim 1, wherein the local device (102) is a smart speaker.

5. The system (100) of claim 1, wherein the local device (102) further comprises a local transmitter (152) configured to wirelessly transmit the personal health voice audio (120) to the remote personal health data ecosystem (104).

6. The system (100) of claim 5, wherein the local transmitter (152) wirelessly transmits the personal health voice audio (120) to the remote personal health data ecosystem (104) via the Internet (148).

7. The system (100) of claim 1, wherein the local device (102) further comprises a voice redirector (154) configured to transmit the non-personal health voice audio (122) to a smart home data ecosystem (156).

8. The system (100) of claim 1, wherein the personal health phrase database (124) comprises a plurality of oral health phrases (158).

## Patentansprüche

1. System (100) zum Verarbeiten von Sprachaudio, umfassend eine lokale Vorrichtung (102), wobei die lokale Vorrichtung (102) Folgendes umfasst:
einen lokalen Sprach-zu-Text-Transkriptor (106), der dazu konfiguriert ist, Sprachtext (108) basierend auf Sprachaudio (110) zu erzeugen, das von einem Benutzer (112) gesprochen wird;
ein lokaler natürlicher Sprachprozessor (NLP) (114), der dazu konfiguriert ist, einen oder mehrere gesprochene Sätze (116) aus dem Sprachtext (108) zu extrahieren; und
einen Maschinenlern-Klassifikator (118), der dazu konfiguriert ist, die Sprachaufnahme (110) basierend auf dem einen oder den mehreren gesprochenen Sätzen (116) und einer Datenbank persönlicher Gesundheitssätze (124) entweder als persönliches Gesundheitssprachaudio (120) oder als nicht-persönliches Gesundheitssprachaudio (122) zu klassifizieren,
wobei das System (100) ferner ein entferntes persönliches Gesundheitsdatenökosystem (104) umfasst, wobei das entfernte persönliche Gesundheitsdatenökosystem (104) Folgendes umfasst:
einen Fernempfänger (126), der dazu konfiguriert ist, das persönliche Gesundheitssprachaudio (120) zu empfangen;
einen entfernten Sprache-zu-Text-Transkriptor (128), der dazu konfiguriert ist, persönlichen Gesundheitssprachtext (130) basierend auf dem persönlichen Gesundheitssprachaudio (120) zu erzeugen;
einen entfernten NLP (132), das dazu konfiguriert ist, einen oder mehrere gesprochene persönliche Gesundheitssätze (134) aus dem persönlichen Gesundheitssprachtext (130) zu extrahieren;
einen Textantwortgenerator (136), der dazu konfiguriert ist, eine Textantwort (138) basierend auf dem einen oder den mehreren gesprochenen persönlichen Gesundheitssätzen (134) zu erzeugen;
einen Text-zu-Sprache-Übersetzer (140), der dazu konfiguriert ist, basierend auf der Textantwort (138) eine Sprachausgabe (142) zu erzeugen; und
einen entfernten Sender (144), der dazu konfiguriert ist, die Sprachantwort (142) drahtlos an einen oder mehrere Lautsprecher (146) zu übertragen, die dazu konfiguriert sind, die Sprachantwort (142) auszugeben.

2. System (100) nach Anspruch 1, wobei das entfernte persönliche Gesundheitsdatenökosystem (104) ferner dazu konfiguriert ist, die Sprachantwort (142) über das Internet (148) an den einen oder die mehreren Lautsprecher (146) zu übertragen.

3. System (100) nach Anspruch 1, wobei das System (100) ferner einen Audiosensor (150) umfasst, der dazu konfiguriert ist, das vom Benutzer (112) gesprochene Sprachaudio (110) aufzunehmen.

4. System (100) nach Anspruch 1, wobei die lokale Vorrichtung (102) ein intelligenter Lautsprecher ist.

5. System (100) nach Anspruch 1, wobei die lokale Vorrichtung (102) ferner einen lokalen Sender (152) umfasst, der dazu konfiguriert ist, das persönliche Gesundheitssprachaudio (120) drahtlos an das entfernte persönliche Gesundheitsdatenökosystem (104) zu übertragen.

6. System (100) nach Anspruch 5, wobei der lokale Sender (152) das persönliche Gesundheitssprachaudio (120) drahtlos über das Internet (148) an das entfernte persönliche Gesundheitsdatenökosystem (104) überträgt.

7. System (100) nach Anspruch 1, wobei die lokale Vorrichtung (102) ferner einen Sprachumleiter (154) umfasst, der dazu konfiguriert ist, das nicht-persönliche Gesundheitssprachaudio (122) an ein Smart-Home-Datenökosystem (156) zu übertragen.

8. System (100) nach Anspruch 1, wobei die persönliche Gesundheitssatzdatenbank (124) eine Vielzahl von Mundgesundheitssätzen (158) umfasst.

## Revendications

1. Système (100) de traitement d'audio vocal comprenant un dispositif local (102), le dispositif local (102) comprenant :
un transcripteur local de parole en texte (106) configuré pour générer un texte vocal (108) à partir de l'audio vocal (110) prononcé par un utilisateur (112) ;
un logiciel local (114) de traitement du langage naturel (NLP) configuré pour extraire une ou plusieurs phrases prononcées (116) à partir du texte vocal (108) ; et
un classificateur d'apprentissage automatique (118) configuré pour classer l'audio vocal (110) comme audio vocal de santé personnelle (120) ou audio vocal de santé non personnelle (122) sur la base d'une ou plusieurs phrases prononcées (116) et d'une base de données de phrases de santé personnelle (124),
dans lequel le système (100) comprend en outre un écosystème de données de santé personnelles à distance (104), l'écosystème de données de santé personnelles à distance (104) comprenant :
un récepteur distant (126) configuré pour recevoir l'audio vocal de santé personnelle (120) ;
un transcripteur à distance de parole en texte (128) configuré pour générer un texte vocal de santé personnelle (130) basé sur l'audio vocal de santé personnelle (120) ;
un NLP distant (132) configuré pour extraire une ou plusieurs phrases prononcées de santé personnelle (134) à partir du texte vocal de santé personnelle (130) ;
un générateur de réponse textuelle (136) configuré pour générer une réponse textuelle (138) basée sur une ou plusieurs phrases prononcées sur la santé personnelle (134) ;
un traducteur de texte en parole (140) configuré pour générer une réponse vocale (142) à partir de la réponse textuelle (138) ; et
un transmetteur distant (144) configuré pour transmettre sans fil la réponse vocale (142) à un ou plusieurs haut-parleurs (146) configurés pour émettre la réponse vocale (142).

2. Système (100) selon la revendication 1, dans lequel l'écosystème de données de santé personnelle à distance (104) est en outre configuré pour transmettre la réponse vocale (142) à un ou plusieurs haut-parleurs (146) via l'Internet (148).

3. Système (100) selon la revendication 1, dans lequel le système (100) comprend en outre un capteur audio (150) configuré pour capturer l'audio vocal (110) prononcé par l'utilisateur (112).

4. Système (100) selon la revendication 1, dans lequel le dispositif local (102) est un haut-parleur intelligent.

5. Système (100) selon la revendication 1, dans lequel le dispositif local (102) comprend en outre un transmetteur local (152) configuré pour transmettre sans fil l'audio vocal de santé personnelle (120) à l'écosystème de données de santé personnelle distant (104).

6. Système (100) selon la revendication 5, dans lequel le transmetteur local (152) transmet sans fil l'audio vocal de santé personnelle (120) à l'écosystème de données de santé personnelle distant (104) via l'Internet (148).

7. Système (100) selon la revendication 1, dans lequel le dispositif local (102) comprend en outre un redirecteur vocal (154) configuré pour transmettre l'audio vocal de santé non personnelle (122) à un écosystème de données de maison intelligent (156).

8. Système (100) selon la revendication 1, dans lequel la base de données de phrases de santé personnelle (124) comprend une pluralité de phrases de santé orale (158).
